# EUROPÄISCHE PATENTSCHRIFT

(11) **EP 0 811 652 B1**
(45) Veröffentlichungstag und Bekanntmachung des Hinweises auf die Patenterteilung: **11.05.2005**
(21) Anmeldenummer: 97109178.0
(22) Anmeldetag: 06.06.1997
(51) Int. Cl.: C08J 3/12, B03C 1/01

(54) **Trockene magnetische Polymerbeadzubereitung**
Dried magnetic composition of polymeric beads
Composition magnétique sèche de perles de polymères

(30) Priorität: 07.06.1996 DE 19622886
(43) Veröffentlichungstag der Anmeldung: 10.12.1997
(73) Patentinhaber: Roche Diagnostics GmbH, 68298 Mannheim (DE)
(72) Erfinder: Harttig, Herbert, Dr., 67122 Altrip (DE)

(56) Entgegenhaltungen:
- EP-A- 0 535 937
- WO-A-94/09368
- US-A- 3 282 150
- US-A- 4 001 360
- US-A- 4 245 026
- US-A- 4 303 784
- DATABASE WPI Section Ch, Week 9218 Derwent Publications Ltd., London, GB; Class A11, AN 92-147680 XP002045058 & JP 04 089 834 A (KANEBO LTD) , 24.März 1992
- PATENT ABSTRACTS OF JAPAN vol. 018, no. 097 (C-1167), 17.Februar 1994 & JP 05 295123 A (JAPAN SYNTHETIC RUBBER CO LTD), 9.November 1993,
- DATABASE WPI Section Ch, Week 7829 Derwent Publications Ltd., London, GB; Class E13, AN 78-52355A XP002045059 & JP 53 065 489 A (LION DENTIFRICE CO LTD) , 10.Juni 1978

## Beschreibung

Gegenstand der Erfindung ist eine trockene magnetische Polymerbeadzubereitung, ein Verfahren zur Herstellung einer solchen Polymerbeadzubereitung, ein Verfahren zur Aufkonzentrierung magnetbeadhaltiger Suspensionen, die Verwendung von Zuckern zur Beschichtung von Polymerbeads, die Verwendung trockener Polymerbeadzubereitungen und Verfahren zum Nachweis eines Analyten mit Hilfe einer trockenen Polymerbeadzubereitung.

Kunststoffbeads werden in analytischen Verfahren schon seit längerem zur Bestimmung von Analyten verwendet. Aus der Bildung eines Agglutinats bzw. der Menge dieses Agglutinats wird meist auf die Anwesenheit des Analyten in der Lösung oder die Menge an Analyt geschlossen.

In jüngerer Zeit werden Kunststoffmagnetbeads in verschiedenen Ausführungsformen als Reagenzien in immunologischen und anderen Testen eingesetzt. Durch die Gegenwart von magnetischen Anteilen sind diese Beads durch Magnetkräfte aus einer Suspension abtrennbar. Unabhängig von der einzelnen Art der Anwendung ist es notwendig, daß die Partikel einzeln vorliegen und keine Aggregate bilden. Bisher wurden solche Polymermagnetbeads ausschließlich in Form von Suspensionen angeboten. Diese Suspensionen werden direkt hergestellt durch Polymerisation von Monomeren. Die entstandenen Partikelchen werden ggf. einer Nachbehandlung, wie z. B. mittels einer anionischen Polymerverbindung (DATABASE WPI, Section Ch, Week 9218, Derwent Publications Ltd., London, GB; class A11, AN92-147680, XP 002045058, 1992) unterzogen, die jedoch stattfindet, ohne die Partikel aus der Suspension zu entfemen. Die in US 4.245.026 beschriebenen magnetischen Partikel werden zur Verhinderung von Aggregaten unter Luft- und Feuchtigkeitsausschluß mit Übergangsmetalcarbonylverbindungen nachbehandelt. Ferner sind aus WO 94/09368 Suspensionen bzw. kolloidale Lösungen von Polymermagnetbeads mit nicht löslichen oder vernetzten Beschichtungen aus Gelatine oder Aminodextran bekannt. Maßnahmen zur Verhinderung von Aggregaten sind in WO 94/09363 nicht angegeben.

Bei Suspensionen dieser Art ergibt sich aber ein spezielles Lagerproblem. Sinken infolge ihrer Schwerkraft die Magnetbeads während der Lagerung auf den Boden der Flasche, so bilden sie dort ein dichtes Depot. Wird während eines Transportvorganges eine solche Flasche auf den Kopf gestellt, so besteht die Möglichkeit, daß das Depot quasi austrocknet.

Bei der Resuspendierung der Magnetbeads ist es nicht möglich, die gebildeten Aggregate wieder vollständig in Einzelpartikel zu zerlegen. Selbst die Anwendung von Ultraschall führt nicht zu befriedigenden Ergebnissen.

Ziel der Erfindung ist die Bereitstellung einer agglomeratfrei resuspendierbaren Polymerbeadzubereitung.

Gegenstand der Erfindung ist eine trockene magnetische Polymerbeadzubereitung wie in Anspruch 1 definiert.

Unter einem Polymerbead wird im Sinne der vorliegenden Erfindung ein Teilchen verstanden, welches partikulär ist und durch Polymerisation von Monomeren hergestellt ist. Bevorzugt haben diese Polymerbeads einen Durchmesser zwischen 0,1 und 100 µm, besonders bevorzugt zwischen 1.0 und 5.0 µm. Die Teilchen haben bevorzugt eine im wesentlichen runde Form. Sie werden auch als Beads bezeichnet, weil ihre Form einer Perle ähnelt. Hauptbestandteil des Beads ist bevorzugt ein organisches Polymer. Hierzu gehören neben den aus einem einzigen Monomere hergestellten Polymer auch die aus mehreren Monomeren hergestellten Polymere, d. h. Copolymere. Als Monomere kommen insbesondere Polystyrol und Polymethylmethacrylat in Frage. Ein Beispiel solcher Polymere sind die sogenannten Latex-Partikel.

Polymerbeads können auf bekannte Weise hergestellt werden, z. B. durch Dispersion der Monomeren in einer Flüssigkeit und Start der Polymerisation je nach Art der Polymerisationsreaktion, d. h. z. B. durch Licht oder Polymerisationsstarter. Die Herstellung von Polymerbeads ist dem Fachmann z. B. aus L.B.Bangs (Amer. Clin. Prod. Rev, 7, 1, 22-26) bekannt.

Magnetbeads sind prinzipiell in Form von Suspensionen käuflich erhältlich, z. B. von der Firma Dynal AS. Es handelt sich um Partikel, die zwar im wesentlichen aus einem organischen Polymeren bestehen, jedoch durch darin eingelagerte magnetische Partikell magnetisch sind. Unter dem Begriff magnetisch sollen nicht nur ferromagnetische, sondern auch para- und superparamagnetische Partikel verstanden werden. Besonders bevorzugt sind die superparamagnetischen Partikel.

Magnetische Polymerbeads können wie in EP-B-0 106 873 beschrieben hergestellt werden.

Manche analytischen Verfahren unter Zuhilfenahme von Polymerbeads verwenden die Polymerbeads zur selektiven Immobilisierung des Analyten bzw. von Analyt-bindenden Stoffen. Für diesen Fall ist es bekannt, die Polymerbeads mit Hilfe von Reagenzien in der Suspension zu modifizieren. Hierzu gehört beispielsweise das Aufbringen einer Schicht von Streptavidin auf die Oberfläche der Partikel. Auf diese Weise können mit Biotin modifizierte Analyten oder analytbindende Substanzen an die Partikeloberfläche gebunden werden.

Gemäß der vorliegenden Erfindung werden diese Partikel nun mit einer Schicht eines leichtlöslichen Stoffes überzogen. Die leichte Löslichkeit dieses Stoffes bezieht sich hierbei auf wäßrige Lösungen. Leicht lösliche Stoffe im Sinne der Erfindung sind für diesen Fall insbesondere Zucker, die einen Schmelzpunkt über den Bedingungen aufweisen, unter denen die trockene Polymerbeadzubereitung hergestellt und gelagert werden soll, bevorzugt von über 100 °C und besonders bevorzugt über Raumtemperatur. Geeignet sind beispielsweise Zucker aus der Reihe der 6-er Zucker und der 6-er-Zucker-Alkohole. Besonders bevorzugt sind Mannit und Trehalose.

Der Überzug der Partikel mit dem löslichen Stoff kann auf vielerlei Art und Weise geschehen. Bevorzugt ist es jedoch, eine Suspension von Polymerbeads, die den leicht löslichen Stoff gelöst enthält, bereitzustellen und diesen unter Bedingungen zu versprühen, bei denen der Suspension die Flüssigkeit entzogen wird. Solche Sprühtrocknungsverfahren sind bekannt. Geht man von den käuflichen Suspensionen aus, genügt die Zugabe einer bestimmten Menge des löslichen Stoffes. Diese sollte so bemessen sein, daß die anwesende Menge des löslichen Stoffes vollständig gelöst ist. Die Menge hängt daher von der Löslichkeit des löslichen Stoffes in der Flüssigkeit der Suspension ab. Im Falle von Zuckern hat sich beispielsweise eine Menge von 0.1 bis 30 Gew.-%, besonders bevorzugt zwischen 1 und 10 Gew.-% als günstig erwiesen. Die Menge an löslichem Stoff muß so bemessen sein, daß die Schicht um jedes einzelne Polymerbead eine Dicke von zwischen 20 nm bis 50 µm, bevorzugt zwischen 0.1 und 5 µm, aufweist. Ob die bei Verwendung einer bestimmten Menge an löslichem Stoff in der Suspension erhaltene Dicke der Beschichtung auf dem Partikel in dem angegebenen Bereich ist, kann sehr leicht mit Hilfe eines Mikroskops gemessen werden. Hierzu werden die Partikel bevorzugt in einen Formkörper eingegossen und Schnitte hergestellt, so daß die Dicke der Partikel und der Beschichtung im Querschnitt abgelesen werden kann. Die solchermaßen hergestellten beschichteten Polymerbeads weisen bevorzugt, unabhängig von ihrer originären Oberflächenstruktur eine glatte Oberfläche und, sofern eine ausreichend dicke Beschichtung gewählt wurde, auch eine kugelige Geometrie auf.

Bevorzugt werden beim Sprühtrocknen vergleichsweise geringe Konzentrationen von magnetischen Polymerbeads in der Suspension eingestellt. Geeignete Konzentrationen sind 0.05 bis 0.5 Gewichts-%. Dann bilden sich überwiegend Tröpfchen ohne oder mit einem einzigen Magnetpartikel. Beim Trocknen im Sprühturm werden dann entsprechend getrocknete Zuckerpartikel bzw. Zuckerpartikel mit im wesentlichen bevorzugt zu mehr als 95 %, höchstens einem einzigen Magnetbead gebildet. Die Ausbildung von Dubletten und Tripletts, d. h. Partikeln, die ein Aggregat von zwei oder drei miteinander verbundenen Magnetpartikeln darstellen, ist sehr deutlich reduziert. Als Flüssigkeit bei der Zubereitung der Polymerbeadsuspension eignen sich insbesondere wäßrige Lösungen. Das Sprühtrocknen wird so lange durchgeführt, bis die erhaltenen Partikel weniger als 1, bevorzugt weniger als 0,1 Gew.-%, freies Wasser enthalten. Ob die Restfeuchte die oben genannten Bedingungen erfüllt, kann auf einfache Weise durch Erhitzung der Produkte des Sprühtrocknungsverfahrens und Ermittlung einer evtl. Gewichtsveränderung festgestellt werden.

Bevorzugt sind die erfindungsgemäßen Polymerbeads schüttfähig.

Überraschenderweise beobachtet man bei Resuspension der nach dem Sprühtrocknen erhaltenen pulverförmigen Masse in einer Flüssigkeit, in der sich der lösliche Stoff löst, in Abwesenheit eines Magnetfeldes praktisch keine Agglomerate von magnetischen Polymerpartikeln. Dasselbe gilt für die nicht-magnetischen Polymerpartikel.

Je nach eingesetzter Menge an löslichem Stoff bilden sich beim Sprühtrocknen neben überzogenen Polymerbeads auch nicht-beadhaltige Partikel, die im wesentlichen aus dem löslichen Stoff bestehen. Im Fall, daß ein zu hoher Anteil an löslichem Stoff stört, z. B. bei analytischen Bestimmungen, ist es möglich, insbesondere die magnetpartikelfreien Zuckerpartikel abzutrennen. Dies ist insbesondere bei Polymermagnetbeads sehr einfach möglich. Hierzu wird eine Suspension der trockenen Polymerbeadzubereitung in einer Flüssigkeit, in der sich der lösliche Stoff gerade nicht löst, z. B. im Fall von Zuckern niederen Alkoholen (1 bis 3 Kohlenstoffatome), z. B. Äthanol, hergestellt. Die Magnetpartikel werden über einen Magneten an der Wandung des Gefäßes, in der sich die Suspension befindet, abgeschieden und die verbleibende Suspension abgetrennt. Nach mehrfachem Waschen mit der Flüssigkeit und Entfernen bzw. Ausschalten des Magneten wird ein Konzentrat von mit dem löslichen Stoff beschichteten polymeren Magnetpartikeln erhalten. Ein Vorteil der Verwendung von niederen Alkoholen ist, daß sie leicht abgetrocknet werden können, so daß ein trockenes Pulver erhalten wird. Resuspendiert man dieses Pulver in der Flüssigkeit, in der sich der lösliche Stoff löst, so erhält man eine Suspension von polymeren Magnetpartikeln, die praktisch frei von Aggregaten und nicht-magnetischen Partikeln ist, insbesondere bevorzugt weniger als 5 % Aggregate enthält.

Ebenfalls Gegenstand der Erfindung ist die Verwendung von Zuckern zur Beschichtung von Polymerbeads.

Ein weiterer Gegenstand der Erfindung ist die Verwendung einer trockenen Polymerbeadzubereitung bei analytischen Verfahren. Als analytische Verfahren werden im Sinne der Erfindung solche Verfahren verstanden, bei denen die Menge eines an eine bestimmte Menge von Polymerbeads gebundenen Analyten bestimmt wird. Solche Verfahren sind dem Fachmann bekannt, z. B. aus L.B.Bangs (Particle based Tests and assays - Pitfalls, Problems and Possibilities in Preparation; Bangs Laboratories, Carmel, IN, USA, October 1990).

Gegenstand der Erfindung ist daher auch ein Verfahren zum Nachweis eines Analyten aus einer Flüssigkeit enthaltend die Schritte Bereitstellung einer trockenen Polymerbeadzubereitung, Resuspension der trockenen Polymerbeads in einer Flüssigkeit, Bindung evtl. anwesender Analyten an die Polymerbeads und Nachweis der polymerbeadgebundenen Analyten. Bevorzugt sind die Polymerbeads wie oben beschrieben in trockenem Zustand, mit einem in der Flüssigkeit löslichen Stoff beschichtet und magnetisch.

Die folgenden Beispiele sollen die vorliegende Erfindung näher erläutern:

### Beispiel 1

### Herstellung trockener und aggregatfrei resuspendierbarer Polymerbeads

25 ml Magnetbeadsuspension M 280, Hersteller Dynal AS, mit einer Konzentration von 10 mg Beads/ml und 3 g D-Mannit und 3 g Wasser wurden miteinander vermischt. Die erhaltene Suspension wurde in einem Niro-Atomizer-Zerstäubungstrockner, Typ Minor, Hersteller Niro, Soeborg, Dänemark, zu einem hellbraunen Pulver versprüht. Die Ausbeute betrug 2,1 g.
Ein kleiner Teil des erhaltenen Pulvers wurde in dest. Wasser resuspendiert. Unter dem Lichtmikroskop waren keine Aggregate erkennbar.

### Beispiel 2

### Aggregation herkömmlicher Partikel

0,5 ml einer Magnetbeadsuspension M 280, Hersteller Dynal AS, wurde bei 40 °C Raumluft getrocknet. Der feste Rückstand wurde aufgenommen, in 10 ml dest. H₂O resuspendiert und 5 Minuten im Ultraschallbad behandelt. Die erhaltene Suspension wies noch große, im Lichtmikroskop deutlich sichtbare, Aggregate auf.

### Beispiel 3

### Aufkonzentrierung trockener und aggregatfreier resuspendierbarer Magnetpolymerbeads

Ca. 50 mg des erhaltenen Pulvers nach Beispiel 1 wurden in einem Eppendorf-Reagenzgefäß mit ca. 1,5 ml Ethanol resuspendiert. An die Wandung des Gefäßes wurde ein Permanentmagnet angelegt. Die Suspension wurde weiß, während sich ein brauner Niederschlag an der Wandung gegenüber dem Magneten bildete. Die verbliebene weiße Suspension wurde abpipettiert und die Waschung mit Isopropanol wiederholt.

Der erhaltene Niederschlag wurde an Raumluft getrocknet.

Das danach vorliegende trockene Pulver wurde in dest. Wasser resuspendiert. Im Lichtmikroskop waren in dieser Suspension keine Aggregate sichtbar.

## Patentansprüche

1. Trockene magnetische Polymerbeadzubereitung enthaltend magnetische Polymerbeads, die eine Beschichtung aus einem wasserlöslichen Stoff enthalten und die Dicke dieser Schicht zwischen 20 nm und 50 µm beträgt.

2. Zubereitung gemäß Anspruch 1 dergestalt, dass der wasserlösliche Stoff ein Zucker ist.

3. Zubereitung gemäß Anspruch 2 dergestalt, dass der Zucker Mannit ist.

4. Zubereitung gemäß Anspruch 1 dergestalt, dass die Polymerbeads einen Durchmesser von zwischen 0,1 und 100 µm haben.

5. Verfahren zur Herstellung einer trockenen magnetischen Polymerbeadzubereitung nach einem der Ansprüche 1-4, enthaltend die Schritte:
- Bereitstellung einer Suspension von magnetischen Polymerbeads, die einen wasserlöslichen Stoff in einer Flüssigkeit enthält,
- Versprühen der Suspension unter Bedingungen, bei denen der Suspension die Flüssigkeiten entzogen wird,
- Einwirkung eines Magnetfeldes auf die Suspension zur Immobilisierung magnetbeadhaltiger Partikel und
- Abtrennung der Flüssigkeit der Suspension mit nicht-magnetbeadhaltigen Partikeln.

6. Verfahren gemäß Anspruch 5 dergestalt, dass die Suspension so versprüht wird, dass zu mehr als 95% Tropfen entstehen, die höchstens ein Polymerbead enthalten.

7. Verfahren gemäß Anspruch 5 dergestalt, dass die magentbeadhaltigen Partikel mit einem wasserlöslichen Stoff beschichtet sind und die Suspensionsflüssigkeit so gewählt wird, dass sich der wasserlösliche Stoff nicht wesentlich darin löst.

8. Verwendung einer trockenen magnetischen Polymerbeadzubereitung nach einem der Ansprüche 1-4 bei analytischen Verfahren.

9. Verfahren zum Nachweis eines Analyten aus einer Flüssigkeit, enthaltend die Schritte:
- Bereitstellung einer trockenen, magnetischen Polymerbeadzubereitung nach einem der Ansprüche 1-4,
- Resuspension der trockenen Polymerbeads in einer Flüssigkeit,
- Bindung des Analyten an Polymerbeads, und
- Nachweis der polymerbeadgebundenen Analyten.

10. Verfahren gemäß Anspruch 9 dergestalt, dass die Polymerbeads in trockenem Zustand mit einem in der Flüssigkeit löslichen Stoff beschichtet sind.

## Claims

1. Dry magnetic polymer bead preparation containing magnetic polymer beads which contain a coating of a water-soluble substance and the thickness of this layer is between 20 nm and 50 µm.

2. Preparation as claimed in claim 1, **characterized in that** the water-soluble substance is a sugar.

3. Preparation as claimed in claim 2, **characterized in that** the sugar is mannitol.

4. Preparation as claimed in claim 1, **characterized in that** the polymer beads have a diameter between 0.1 and 100 µm.

5. Process for producing a dry magnetic polymer bead preparation as claimed in one of the claims 1 - 4 comprising the steps:
- preparing a suspension of magnetic polymer beads which contains a water-soluble substance in a liquid,
- spraying the suspension under conditions in which the liquids are removed from the suspension,
- allowing a magnetic field to act on the suspension to immobilize particles containing magnetic beads and
- separating the liquid of the suspension containing particles that do not contain magnetic beads.

6. Process as claimed in claim 5, **characterized in that** the suspension is sprayed in such a manner that more than 95 % of the drops that are formed contain no more than one polymer bead.

7. Process as claimed in claim 5, **characterized in that** the particles containing magnetic beads are coated with a water-soluble substance and the suspension liquid is selected such that the water-soluble substance does not substantially dissolve in it.

8. Use of a dry magnetic polymer bead preparation as claimed in one of the claims 1 - 4 in analytical methods.

9. Method for detecting an analyte from a liquid comprising the steps:
- preparing a dry magnetic polymer bead preparation as claimed in one of the claims 1 - 4,
- resuspending the dry polymer beads in a liquid,
- binding the analyte to polymer beads and
- detecting the polymer bead-bound analyte.

10. Method as claimed in claim 9, **characterized in that** the polymer beads are coated in a dry state with a substance that is soluble in the liquid.

## Revendications

1. Composition sèche, magnétique de perles de polymère contenant des perles magnétiques de polymère, qui contiennent un revêtement en une substance soluble dans l'eau et l'épaisseur de cette couche est comprise entre 20 nm et 50 µm.

2. Composition selon la revendication 1, dans laquelle la substance soluble dans l'eau est un sucre.

3. Composition selon la revendication 2, dans laquelle le sucre est le mannitol.

4. Composition selon la revendication 1, dans laquelle les perles de polymère présentent un diamètre compris entre 0,1 et 100 µm.

5. Procédé pour la préparation d'une composition sèche, magnétique de perles de polymère selon l'une quelconque des revendications 1 à 4, présentant les étapes de :
- mise à disposition d'une suspension de pertes magnétiques de polymère qui contient une substance soluble dans l'eau dans un liquide,
- pulvérisation de la suspension dans des conditions dans lesquelles les liquides sont éliminés de la suspension,
- action d'un champ magnétique sur la suspension pour immobiliser les particules contenant les perles magnétiques et
- séparation du liquide de la suspension avec les particules ne contenant pas de perles magnétiques.

6. Procédé selon la revendication 5, dans lequel la suspension est pulvérisée de telle manière qu'il se forme plus de 95% de gouttes qui contiennent au plus une perle de polymère.

7. Procédé selon la revendication 5, d ans lequel les particules contenant les perles magnétiques sont revêtues d'une substance soluble dans l'eau et le liquide de la suspension est choisi de telle manière que la substance soluble dans l'eau ne s'y dissout pas de manière essentielle.

8. Utilisation d'une composition sèche, magnétique de perles de polymère selon l'une quelconque des revendications 1 à 4 dans des procédés analytiques.

9. Procédé pour I a détection d'un analyte d ans u n liquide, p résentant les étapes de :
- mise à disposition d'une composition sèche, magnétique de perles de polymère selon l'une quelconque des revendications 1 à 4,
- remise en suspension des perles sèches de polymère dans un liquide,
- fixation de l'analyte sur les perles de polymère, et
- détection de l'analyte lié sur les perles de polymère.

10. Procédé selon la revendication 9, dans lequel les perles de polymère sont revêtues à l'état sec par une substance soluble dans le liquide.
